# EUROPEAN PATENT SPECIFICATION

(11) **EP 1 916 007 B1**
(45) Date of publication and mention of the grant of the patent: **01.07.2009**
(21) Application number: 06255364.9
(22) Date of filing: 18.10.2006
(51) Int. Cl.: A61L 31/12, A61L 27/10, A61L 27/40

(54) **Aluminium oxide coated implants and components**
Aluminiumoxid-beschichtete Implantate und Komponente
Implants et composants revêtu avec de l'oxyde d'aluminium

(43) Date of publication of application: 30.04.2008
(73) Proprietor: DePuy Products, Inc., Warsaw, IN 46581 (US)
(72) Inventor: Overholser, Ronald, Columbia City, IN 46725 (US); Smith, Bryan, Fort Wayne, IN 46804 (US)
(74) Representative: Belcher, Simon James

(56) References cited:
- EP-A- 1 616 978
- EP-A2- 0 248 117
- WO-A-92/17623
- WO-A-99/24634
- DE-A1- 4 422 686
- US-A1- 2003 008 764
- DATABASE WPI Week 199630 Derwent Publications Ltd., London, GB; AN 1996-294774 XP002423789 & JP 08 126695 A (KYOCERA CORP) 21 May 1996 (1996-05-21)

## Description

This invention relates to orthopaedic joint prosthesis components and to a method of making such components.

Interest in medical prosthetic implants with longer lifespans has increased due to demographic shifts in the population of medical implant recipients. As in the general population, the life expectancy of medical implant recipients has been steadily increasing. Additionally, the number of younger and more active implant recipients has also been increasing. Each of these trends highlights the importance of longer-lasting, high performance orthopaedic implants that are less likely to require replacement, i.e., revision, during a recipient's lifetime.

Thus, of concern is that such longer-lasting implants be able to withstand years of wear and the variety of stresses associated with being implanted in a body. Joint replacement implants must be strong enough to tolerate changing mechanical loads, while at the same time, transferring portions of the loads to surrounding bone tissue. Another requirement is that the bearing (i.e., articulating) surfaces of joint replacement implants must be resistant to a corrosive environment and withstand body and surface contact forces and adhesive and abrasive wear processes that are associated with movement of the joint. Mechanical and physical properties suitable for withstanding years of mechanical loading and wear can be difficult to find in a single material.

Currently, metals, including metal alloys, are used as structural material for medical implants. Metal alloys such as steel, cobalt alloys, and titanium alloys exhibit a range of strengths, hardness, and fatigue resistance. Moreover, these metals can be formed by metalworking processes and machined. Commonly, implants include a metal bearing surface configured to move against a second bearing surface composed of a low-friction polymer, such as ultra high molecular weight polyethylene (UHMWPE), or another metal component. Particles and debris have been known to dissociate from these implants due to wear processes once implanted in a recipient's body. These dissociated particles are problematic, as those that become disposed in surrounding tissue have been linked to inflammation and degradation of surrounding tissue. This inflammatory reaction can lead to joint pain and loosening of the implant. Eventually, the condition can require removal and replacement of the implant in a complicated and troublesome revision procedure. Hard particles such as cement particles (in devices implanted with cement) or delaminated metal from an implant's porous or textured fixation surface can become lodged in the articulating surface of a polymeric implant or trapped between opposing articulating surfaces and scratch the previously smooth metallic surface, and thereby reduce implant performance, increase abrasive wear of the articulating counterface, and even lead to delamination of other surface material.

Previous efforts to reduce the wear of metallic implant surfaces have included surface modifications such as ion implantation, gas nitriding, and high temperature oxidation. Each approach can have limitations. For example, generally, the peak hardness of surfaces obtained by ion implantation and nitriding is not as high as the peak hardness that can be obtained by other surface modifications such as ceramic overlay coatings. Ion implantation approaches can also have theoretical or economic limits on the depth of the surface modified material. Substrates underlying surface layers created by high temperature oxidation (for example, Zr-2.5Nb alloy) are not as strong or hard as other substrate metals (for example, CoCrMo or titanium alloys) and may not be compatible with conventional processes for creating porous tissue ingrowth surface structures.

In a different approach, bulk metal substrates in implants have been replaced with bulk ceramics, such as in certain hip implants that include bulk oxide ceramic femoral heads. Such ceramics do not corrode in the body, are wear-resistant, and they can withstand large compressive loads. However, bulk ceramics tend to be stiffer and more brittle than metals. Reports have shown that some bulk ceramic implant components are prone to catastrophic fracture and, thus, may require immediate revision.

To obtain the beneficial wear properties of bulk ceramics without the risk of fracture, there have been attempts to apply ceramic coatings to the articulating surface of a metal implant. The properties of these ceramic coatings are quite varied, depending on the ceramic type and manner of application. However, the development of implants coated with particular ceramics (for example, aluminium oxide) has been limited. Techniques for applying particular ceramic coatings have resulted in coatings that are alternately too thin, porous, unstable in the body, or poorly adhered to the underlying metal substrate for use on orthopaedic bearing surfaces.

EP-A-248117 discloses an orthopaedic joint prosthesis component having a body formed from titanium, which has an outer coating provided by TiO₂, Al₂O₃, ZrO₂ or Cr₂O₃, and an intermediate layer of titanium between the body and the outer coating which can improve adherence.

The invention provides an orthopaedic joint prosthesis component having a bearing surface, as defined in claim 1.

The invention also provides a method of making an orthopaedic joint prosthesis component having a bearing surface, as defined in claim 9.

Suitable medical implant devices and components thereof include, but not limited to, orthopaedic prostheses for the hip, knee, ankle, shoulder, elbow, and spine. Exemplary medical implant devices include a full or partial knee arthroplasty prosthesis, full or partial hip arthroplasty prosthesis, full or partial elbow arthroplasty prosthesis, full or partial wrist arthroplasty prosthesis, full or partial shoulder arthroplasty prosthesis, full or partial ankle arthroplasty prosthesis, and full or partial articulating spinal segment arthroplasty prosthesis. Exemplary components of medical implant devices include a femoral component (for example, for replacing one or more femoral condyles) or a tibial component (for example, for replacing at least a portion of a proximal tibial plateau) of a knee prosthesis (for example, a uni-compartmental or total knee arthroplasty prosthesis), a femoral component (for example, for replacing at least the proximal portion or head of the femur) or an acetabular cup (for example, for replacing the hip bone's femoral socket) of a hip prosthesis, a humeral component (for example, for replacing the distal portion of the humerus) or an ulnar component (for example, for replacing the proximal portion of the ulna) of an elbow prosthesis, a metacarpal component (for replacing at least a portion of one or more metacarpal bones) or radial component (for replacing the distal portion of the radius) of a wrist prosthesis, a humeral component (for example, for replacing the proximal portion or head of the humerus) or glenoid component (for example, for replacing the glenoid or socket portion of the scapula) of a shoulder prosthesis, a tibial component (for example, for replacing the distal portion of the tibia) or talar component (for example, for replacing the proximal portion of the talus) of an ankle prosthesis, and an endplate component (for example, for contacting the superior or inferior portion of a cervical, lumbar or thoracic vertebra) or spacer component (for example for insertion between endplate components) of a vertebral disc prosthesis.

The metal substrate can be any suitable metal substrate other than steel. Unless otherwise indicated herein, the term "metal" refers to pure metals and metal alloys. The "metal substrate" can be the entire, or nearly the entire, structure that substantially forms the medical implant device or component thereof; or the "metal substrate" can be a portion of the structure that substantially forms the medical implant device or component thereof, with the remainder of the structure that substantially forms the medical implant device or component thereof comprising other material. When the metal substrate is a portion of the structure that substantially forms the medical implant device or component thereof, the metal substrate can be the entire surface of, or a portion of the surface of, the structure that substantially forms the medical implant device or component thereof.

While the metal substrate can be any suitable portion of the medical implant device or component thereof, the metal substrate desirably includes a bearing surface. As used herein, a "bearing surface" refers to a portion of the medical implant device or component thereof that is configured to articulate with or move against a second surface when the medical implant device or component thereof is implanted in the body of a patient. The second surface can be bone, cartilage, or other material that is native to the body. More commonly, the second surface is another, i.e., a second, bearing surface in a medical implant device or component thereof. For example, in certain embodiments of the invention, a coated bearing surface (comprising a substrate, an intermediate coating, and an outer coating of aluminium oxide) is designed to bear against a second bearing surface of the same or different medical implant device or component thereof, wherein the second bearing surface can comprise any suitable material, for example, metal, ceramic, polymer, or combinations thereof. Typically, the second bearing surface comprises a polymer such as an ultra high molecular weight polyethylene (UHMWPE), which is widely used as a polymeric bearing surface, for example, in knee implants and hip implants. The second bearing surface may also comprise another coated bearing surface or an uncoated metallic bearing surface.

Examples of bearing surfaces to which the present invention is applicable include (a) the surface at the distal end of a femoral component of a knee prosthesis, which surface articulates with a second bearing surface on the tibial component of the prosthesis, (b) the surface of a femoral head component of a hip prosthesis, which surface articulates against the second bearing surface of the acetabular cup component of the prosthesis, (c) an articulating surface of an elbow arthroplasty prosthesis, for example, the surface of the distal end of the humoral component or the surface of the proximal end of the ulnar component, which surfaces articulate against each other, (d) the surface of a humoral head component of a shoulder prosthesis, which surface articulates against the glenoid component of the prosthesis, (e) the surface of a talus component of an ankle prosthesis, which surface articulates against the tibial component of the arthroplasty, and (f) the surface of a spinal disc replacement prosthesis' endplate component, which surface articulates against another endplate or the core/spacer component disposed between two endplates of the prosthesis.

The metal substrate can comprise, consist essentially of, or consist of any suitable metal, desirably a biocompatible metal. Desirable metals include metals with suitable mechanical properties for use in joint replacement prostheses. The metal preferably does not readily corrode in a patient into which the medical implant device or component thereof is intended to be placed, and preferably possesses appropriate strength and fatigue characteristics. Exemplary preferred metal substrates include cobalt, cobalt alloys, titanium, titanium alloys, and mixtures of these. Suitable cobalt-chromium alloys include, but are not limited to, the cast, forged, and wrought cobalt-28-chromium-6-molybdenum (Co28Cr6Mo) alloys described in, for example, ASTM Standards F75-01, F799-02, and F1537-00, respectively. Suitable titanium-aluminium alloys include, but are not limited to, the titanium-3-aluminium-2.5-vanadium alloy (Ti-3Al-2.5V) described in, for example, ASTM Standard F2146-01 and the titanium-6-aluminium-4-vanadium (Ti-6Al-4V) alloy described in, for example, ASTM Standard F136-02a. ASTM standards are available in print or electronic media from ASTM International (West Conshohocken, PA).

An intermediate coating of a material other than aluminium oxide is adhered to the metal substrate. The material of the intermediate coating preferably has one or more physical or chemical properties (for example a thermal expansion coefficient, elastic modulus, crystal structures, and/or chemical compatibility) intermediate between the physical or chemical properties of the metal substrate and the outer coating of aluminium oxide, so as to provide a transition gradient of those properties on the surface of the medical implant device or component thereof. Through this transition gradient of mechanical and physical properties, the intermediate coating can provide a desirable level of adhesion between the metal substrate and the outer coating of aluminium oxide.

The intermediate coating can be of any suitable thickness. The intermediate coating desirably has a thickness of about 0.001 µm or more , for example, about 0.01 µm or more, 0.05 µm or more, about 0.1 µm or more, or about 0.2 µm, or about 0.4 µm or more. The intermediate coating desirably has a thickness of about 10 µm or less, for example, about 5 µm or less, about 2 µm or less, or about 1 µm or less.

The intermediate coating is adhered to the metal substrate by any suitable technique including physical vapour deposition (PVD), chemical vapour deposition (CVD), and thermal spraying deposition (for example, plasma spraying). Generally, PVD is preferred because of its ability to produce high quality, dense coatings of many metals and ceramic compounds.

The intermediate coating can constitute one or more layers, which can be separately applied by any suitable technique and adhered to each other. Each layer desirably comprises the material(s) described herein for the intermediate coating and is applied by the techniques described herein for the intermediate coating.

One or more of the following considerations desirably is taken into account when selecting the material and thickness of the intermediate coating. The material and thickness of the intermediate coating preferably promotes the adherence of the outer coating of aluminium oxide to the metal substrate. Thus, the material and thickness of the outer coating desirably improve the indirect adherence of the outer coating to the metal substrate relative to the adherence of the outer coating directly to the same substrate (i.e., without the intermediate coating). Common methods for assessing the adhesion of ceramic coatings on metals include scratch and indentation tests. Suitable tests are described in, for example, Steinmann et al., Thin Solid Films 154;333-349 (1987), Jindal et al., Thin Solid Films 154:361-375 (1987), Vidakis et al., Journal of Materials Processing Technologies 143-144:481-485(2003), and the German standard VDI 3198. Desirably, the material and thickness of the intermediate coating provides one or more physical or chemical properties (for example a thermal expansion coefficient, elastic modulus, crystal structures, and/or chemical compatibility) intermediate between the physical or chemical properties of the metal substrate and the outer coating of aluminium oxide, so as to provide a transition gradient of those properties on the surface of the medical implant device or component thereof. Also desirably, the material and thickness of the intermediate coating increase the outer coating's resistance to wear and shear stress (relative to the outer coating's resistance without the intermediate coating) during production and/or after implantation in a body of a patient.

A coating of aluminium oxide (also known as Al₂O₃ or alumina) is adhered onto at least a portion of the intermediate coating. The outer coating is so named because it is outermost on the medical implant device or component thereof relative to the metal substrate and the intermediate coating disposed between the metal substrate and the outer coating. The aluminium oxide can be any suitable aluminium oxide. Desirably, the aluminium oxide is in a biocompatible and thermodynamically-stable α-phase, having a crystalline structure with hexagonal close-packing oxygen atoms and aluminium atoms occupying two thirds of the octahedral lattice interstices. The outer coating preferably includes a layer of single phase α-aluminium oxide. The α-phase is distinguished from the metastable κ-phase and γ-phase aluminium oxides. These less stable forms of aluminium oxide are reportedly more likely to solubilise when disposed in a body of a patient. See, for example, Toni et al., J. Arthroplasty, 9: 4 (1994).

The outer coating desirably has a thickness of about 1 µm or more, for example, about 1.5 µm or more, about 2 µm or more, about 2.5 µm or more, about 3 µm or more, about 3.5 µm or more, or about 4 µm or more, or about 5 µm or more. In general, the upper limit of the thickness of the outer coating is determined by considerations that can, but do not necessarily, include production cost, coating adhesion, residual stresses, scratch and indentation resistance, and substrate fatigue resistance and coating fatigue resistance. The outer coating typically has a thickness of about 100 µm or less, about 50 µm or less, about 40 µm or less, about 30 µm or less, about 20 µm or less, or about 10 µm or less. The outer coating desirably has a thickness of about 15 µm or less, for example, about 12 µm or less, about 10 µm or less, or about 9 µm or less, or about 8 µm or less.

The outer coating is disposed on, and adhered to, the intermediate coating by physical vapour deposition (PVD) onto at least a portion of the intermediate coating. PVD techniques include ion plating, arc discharge evaporation, activated reactive evaporation (ARE), and magnetron sputtering techniques. PVD aluminium oxide films have been deposited by reactive evaporation and reactive sputtering of a metallic aluminium target in an oxygen environment and direct sputtering of an oxide target. Preferred PVD techniques include ionized magnetron sputtering, pulsed magnetron sputtering, RF magnetron sputtering and AC magnetron sputtering. As used herein, PVD techniques do not include ion beam assisted deposition (IBAD). PVD equipment and services are available commercially, for example, from Teer Coatings Ltd (Droitwich, Worcestershire, England) and Balzers AG, Principality of Liechtenstein. Moreover, PVD techniques are described in Seino et al., J. Vac. Sci. Tech., A20: 634-637 (2002), Kelly et al., J. Vac. Sci. Tech., A17: 945-953 (2002), Zywitzki et al., Surface & Coatings Tech., 94-95: 303-308 (1997), Zywitzki et al., Surface & Coatings Tech., 86-87: 640-647 (1996), and US-6210726.

PVD typically can be used at relatively low temperatures. Although the formation of the stable phase aluminium oxide coating requires heating of the substrate to be coated, the required temperatures are typically less than with thermal CVD, where phase aluminium oxide coatings are formed above 1000°C (Prengel et al., Surf. Coat. Tech., 68-69:217 (1994)). Thus, for example, a stable α-phase aluminium oxide coating can be applied by PVD using temperatures of less than about 1000°C, for example, less than about 950°C, less than about 900°C, or less than about 850°C. Using PVD, an α-phase aluminium oxide coating can be applied at temperature ranges between about 500°C and about 850°C, for example, between about 700°C and about 800°C, between about 600 °C and about 700°C, or between about 500°C and 600°C. Reduced temperatures used for PVD also allow optimization of the mechanical properties of the coating/substrate system.

PVD can be used to produce very fine-grained aluminium oxide coatings with fine surface finishes that are advantageous for bearing applications. The bearing surfaces of medical implant devices or components thereof often need to be polished to minimize friction between bearing surfaces and the wear rates of a bearing couple. A fine grained aluminium oxide coating that requires less or no post-coating polishing can be advantageous, as imperfect polishing can cause surface defects in the coating microstructure and contribute to non-uniform coating thicknesses on an implant, both of which could reduced performance.

The invention also provides a method for making the medical implant device or component thereof of the invention. The method of comprises (i) providing a medical implant device or component thereof that comprises a metal substrate other than steel, the substrate comprising a surface, (ii) applying an intermediate coating of a material other than aluminium oxide to the substrate surface, and (iii) depositing an outer coating of aluminium oxide by physical vapour deposition on to at least a portion of the intermediate coating. The various elements of the method are as previously described in the context of the medical implant device or component thereof of the invention.

## Claims

1. An orthopaedic joint prosthesis component having a bearing surface, which comprises:
(a) a metal substrate other than steel which provides a surface,
(b) an intermediate coating adhered to the substrate surface, and
(c) an outer coating of aluminium oxide on at least a portion of the intermediate coating to provide the bearing surface,
**characterised in that** the intermediate coating comprises a material selected from the group consisting of titanium aluminium nitride (TiAIN), chromium aluminium nitride (CrAIN), aluminium nitride (AlN), titanium carbonitride (TiCN), titanium nitride (TiN), chromium oxide (Cr₂O₃), titanium aluminide (TiAl), chromium nitride (CrN), and combinations thereof, and the outer coating is deposited on the intermediate coating by physical vapour deposition and has a thickness of not more than 50 µm.

2. The component of claim 1, in which the outer coating comprises α-aluminium oxide.

3. The component of claim 1, in which the metal substrate comprises a biocompatible metal or metal alloy selected from the group consisting of cobalt, cobalt alloys, titanium, titanium alloys, and mixtures thereof.

4. The component of claim 1, in which the metal substrate comprises cobalt chromium alloy or a titanium alloy.

5. The component of claim 1, in which the metal substrate comprises Co-28Cr-6Mo, Ti-3Al-2.5V, or Ti-6Al-4V.

6. The component of claim 1, in which the device is a prosthetic device for use in a knee joint, hip joint, elbow joint, shoulder joint, ankle joint, or an articulating vertebral segment.

7. The component of claim 1, in which the device is a knee or hip replacement prosthesis that comprises a femoral portion, the femoral portion comprises a bearing surface for contacting (i) a tibial proximal articular surface or (ii) an acetabular cup, and at least a portion of the bearing surface is the substrate surface.

8. The component of claim 7, in which the metal substrate comprises Co-28Cr-6Mo, Ti-3Al-2.5V, or Ti-6Al-4V.

9. The component of claim 1, in which the outer coating has a thickness of not more than 15 µm.

10. A method of making an orthopaedic joint prosthesis component having a bearing surface, the method comprising:
(i) providing a component thereof that comprises a metal substrate other than steel, the substrate comprising a surface,
(ii) applying an intermediate coating to the substrate surface of a material selected from the group consisting of titanium aluminium nitride (TiAIN), chromium aluminium nitride (CrAIN), aluminium nitride (AlN), titanium carbonitride (TiCN), titanium nitride (TiN), chromium oxide (Cr₂O₃), titanium aluminide (TiAl), chromium nitride (CrN), and combinations thereof, and
(iii) depositing an outer coating of aluminium oxide having a thickness of not more than 50 µm by physical vapour deposition on to at least a portion of the intermediate coating to provide the bearing surface.

11. The method of claim 10, in which the outer coating has a thickness of not more than 15 µm.

## Patentansprüche

1. Eine orthopädische Gelenkprothesenkomponente mit einer Lagerfläche, umfassend:
a) ein Metallsubstrat mit Ausnahme von Stahl, welches eine Oberfläche bereitstellt,
b) eine zwischenliegende Beschichtung, die an der Substratoberfläche haftet und
c) eine äußere Beschichtung aus Aluminiumoxid auf wenigstens einem Abschnitt der zwischenliegenden Beschichtung, um die Lagerfläche bereitzustellen,
**dadurch** charakterisiert, das die zwischenliegende Beschichtung ein Material umfasst, das aus der Gruppe bestehend aus Titanaluminiumnitrid (TiAlN), Chromaluminiumnitrid (CrAlN), Aluminiumnitrid (AlN), Titankohlenstoffnitrid (TiCN), Titannitrid (TiN), Chromoxid (Cr₂O₃), Titanaluminid (TiAl), Chromnitrid (CrN) und Kombinationen derselben ausgewählt ist, und die äußere Beschichtung auf der zwischenliegenden Beschichtung durch physikalische Gasphasenabscheidung angeordnet ist und eine Dicke von nicht mehr als 50 µm aufweist.

2. Komponente nach Anspruch 1, bei welcher die äußere Beschichtung α-Aluminiumoxid aufweist.

3. Komponente nach Anspruch 1, bei welcher das Metallsubstrat ein biokompatibles Metall oder eine Metalllegierung aufweist, die aus der Gruppe bestehend aus Kobalt, Kobaltlegierungen, Titan, Titanlegierungen und Mischungen derselben ausgewählt ist.

4. Komponente nach Anspruch 1, bei welcher das Metallsubstrat Kobaltchromlegierung oder eine Titanlegierung aufweist.

5. Komponente nach Anspruch 1, bei welcher das Metallsubstrat Co-28Cr-6Mo, Ti-3Al-2.5V oder Ti-6Al-4V aufweist.

6. Komponente nach Anspruch 1, bei welcher die Vorrichtung eine Prothesenvorrichtung zur Verwendung in einem Kniegelenk, Hüftgelenk, Ellbogengelenk, Schultergelenk, Knöchelgelenk oder ein in einer Gelenkverbindung stehendes vertebrales Segment ist.

7. Komponente nach Anspruch 1, bei welcher die Vorrichtung eine Knie- oder Hüftersatzprothese ist, die einen Oberschenkelabschnitt aufweist, wobei der Oberschenkelabschnitt eine Lagerfläche zum Kontaktieren (I) einer proximalen in einer Gelenkverbindung stehenden Schienbeinoberfläche oder (II) einer azetabularen Schale aufweist, und wenigstens ein Abschnitt der Lagerfläche in der Substratoberfläche liegt.

8. Komponente nach Anspruch 7, bei welcher das Metallsubstrat Co-28Cr-6Mo, Ti-3Al-2.5V oder Ti-6Al-4V aufweist.

9. Komponente nach Anspruch 1, bei welcher die äußere Beschichtung eine Dicke von nicht mehr als 15 µm aufweist.

10. Ein Verfahren zur Herstellung einer orthopädischen Gelenkprothesenkomponente mit einer Lagerfläche, wobei das Verfahren umfasst:
(I) Bereitstellen einer Komponente derselben, die ein Metallsubstrat mit Ausnahme von Stahl umfasst, wobei das Substrat eine Oberfläche aufweist,
(II) Applizieren einer zwischenliegenden Beschichtung auf die Substratoberfläche aus einem Material, das aus der Gruppe bestehend aus Titanaluminiumnitrid (TiAlN), Chromaluminiumnitrid (CrAlN), Aluminiumnitrid (AlN), Titankohlenstoffnitrid (TiCN), Titannitrid (TiN), Chromoxid (Cr₂O₃), Titanaluminid (TiAl), Chromnitrid (CrN) und Kombinationen derselben ausgewählt ist, und
(III) Anordnen einer Beschichtung aus Aluminiumoxid, die eine Dicke von nicht mehr als 50 µm aufweist, durch physikalische Gasphasenabscheidung auf wenigstens einen Abschnitt der zwischenliegenden Beschichtung, um die Lagerfläche bereitzustellen.

11. Verfahren nach Anspruch 10, bei welchem die äußere Beschichtung eine Dicke von nicht mehr als 15 µm aufweist.

## Revendications

1. Composant de prothèse orthopédique pour articulation ayant une surface d'appui, qui comprend :
(a) un substrat en métal différent de l'acier qui fournit une surface,
(b) un revêtement intermédiaire collé sur la surface de substrat, et
(c) un revêtement externe en oxyde d'aluminium sur au moins une partie du revêtement intermédiaire pour fournir la surface d'appui,
**caractérisé en ce que** le revêtement intermédiaire comprend un matériau choisi dans le groupe constitué par le nitrure d'aluminium titane (TiAlN), le nitrure d'aluminium chrome (CrAlN), le nitrure d'aluminium (AlN), le carbonitrure de titane (TiCN), le nitrure de titane (TiN), l'oxyde de chrome (Cr₂O₃), l'aluminure de titane (TiAl), le nitrure de chrome (CrN), et leurs combinaisons, et le revêtement externe est déposé sur le revêtement intermédiaire par dépôt de vapeur physique et a une épaisseur d'au plus 50 µm.

2. Composant selon la revendication 1, dans lequel le revêtement externe comprend de l'oxyde d'aluminium α.

3. Composant selon la revendication 1, dans lequel le substrat en métal comprend un métal ou alliage de métal biocompatible choisi dans le groupe constitué par le cobalt, les alliages de cobalt, le titane, les alliages de titane et leurs mélanges.

4. Composant selon la revendication 1, dans lequel le substrat en métal comprend un alliage de chrome et de cobalt ou un alliage de titane.

5. Composant selon la revendication 1, dans lequel le substrat en métal comprend Co-28Cr-6Mo, Ti-3Al-2,5V ou Ti-6Al-4V.

6. Composant selon la revendication 1, dans lequel le dispositif est un dispositif prothétique destiné à être utilisé dans une articulation du genou, une articulation de la hanche, une articulation du coude, une articulation de l'épaule, une articulation de la cheville ou un segment vertébral d'articulation.

7. Composant selon la revendication 1, dans lequel le dispositif est une prothèse de remplacement de genou ou de hanche qui comprend une partie fémorale, la partie fémorale comprend une surface d'appui pour entrer en contact avec (i) une surface articulaire proximale tibiale ou (ii) une cupule acétabulaire, et au moins une partie de la surface d'appui est la surface de substrat.

8. Composant selon la revendication 7, dans lequel le substrat en métal comprend Co-28Cr-6Mo, Ti-3Al-2,5V ou Ti-6Al-4V.

9. Composant selon la revendication 1, dans lequel le revêtement externe a une épaisseur d'au plus 15 µm.

10. Procédé pour fabriquer un composant de prothèse orthopédique pour articulation ayant une surface d'appui, le procédé comprenant les étapes consistant à :
(i) prévoir son composant qui comprend un substrat en métal différent de l'acier, le substrat comprenant une surface,
(ii) appliquer un revêtement intermédiaire sur la surface de substrat d'un matériau choisi dans le groupe constitué par le nitrure d'aluminium titane (TiAlN), le nitrure d'aluminium chrome (CrAlN), le nitrure d'aluminium (AlN), le carbonitrure de titane (TiCN), le nitrure de titane (TiN), l'oxyde de chrome (Cr₂O₃), l'aluminure de titane (TiAl), le nitrure de chrome (CrN), et leurs combinaisons, et
(iii) déposer un revêtement externe d'oxyde d'aluminium ayant une épaisseur d'au plus 50 µm par dépôt de vapeur physique sur au moins une partie du revêtement intermédiaire pour fournir la surface d'appui.

11. Procédé selon la revendication 10, dans lequel le revêtement externe a une épaisseur d'au plus 15 µm.
